# EUROPÄISCHE PATENTSCHRIFT

(11) **EP 0 637 433 B1**
(45) Veröffentlichungstag und Bekanntmachung des Hinweises auf die Patenterteilung: **29.09.1999**
(21) Anmeldenummer: 94810436.9
(22) Anmeldetag: 22.07.1994
(51) Int. Cl.: A61B 17/14, B23D 61/02, B23D 61/12

(54) **Sägeblatt für oszillierend oder rotierend ausgeführte Trennschnitte**
Saw blade for making a cut by oscillation or rotation
Lame de scie pour effectuer une coupe par oscillation ou par rotation

(30) Priorität: 03.08.1993 CH 232793
(43) Veröffentlichungstag der Anmeldung: 08.02.1995
(73) Patentinhaber: Ricana AG, 8045 Zürich (CH)
(72) Erfinder: Arnegger, Richard E., CH-8713 Uerikon (CH); Gunnewijk, Antonius G. M., CH-8714 Feldbach (CH); Maurer, Thomas, CH-8044 Zürich (CH)
(74) Vertreter: Ritscher, Thomas, Dr.

(56) Entgegenhaltungen:
- EP-A- 0 382 483
- WO-A-93/01751
- DE-A- 1 577 984
- DE-A- 2 849 760
- DE-A- 3 222 339
- GB-A- 2 107 641
- US-A- 1 967 020
- US-A- 3 910 774

## Beschreibung

Die Erfindung betrifft ein Sägeblatt für oszillierend oder rotierend ausgeführte Trennschnitte mit einem Schneidbereich, einem nichtschneidenden Blattbereich und einem Halterungsbereich, insbesondere mit Sägezähnen als Schneidbereich und zur Anwendung in der Knochenchirurgie, sowie ein Herstellverfahren. Derartige Sägeblätter, oder allgemein ausgedrückt auch blattförmige Trennwerkzeuge mit einem peripheren schneidenden Spanbereich, werden beispielsweise für medizinische Anwendungen oder auch in der Feinwerktechnik, im Flugzeugbau, im Bootsbau, in der Mikroelektronik oder zum Trennen von Verbundwerkstoffen eingesetzt. Für die besonders anspruchsvolle und heikle Anwendung in der Knochenchirurgie sind z.B. Mikrosägen mit geschränkten Zähnen oder Weiterentwicklungen mit ungeschränktem Zahnbereich gemäss der DE PS 32 22 339 bekannt. Diese bekannten Trennwerkzeuge weisen aber immer noch erhebliche Nachteile auf. Es handelt sich meist um kleine Werkzeuge für feine Arbeiten, die z.B. mittels Ätztechnik oder Lasertechnik hergestellt werden können. Eine besonders einfache und rationelle Herstellung von Sägeblättern mittels Ätztechnik hat jedoch die Entstehung von Poren und Unebenheiten an der Oberfläche zur Folge. Eine unebene und Poren aufweisende Oberfläche ist jedoch für chirurgische Anwendungen, welche absolut keimfrei ausgeführt werden müssen, sehr nachteilig, da sich in den Unebenheiten Keime und Bakterien festsetzen können, was die erforderliche Sterilisation und die Keimfreihaltung sehr erschwert. Ein weiterer Nachteil stellt die mangelhafte Gleitfähigkeit bisheriger Sägeblätter dar. Von grösster Bedeutung bei anspruchsvollen Trennschnitten in heiklen Materialien und vor allem in der Knochenchirurgie ist es, die Reibung am Material zu verringern und damit möglichst wenig Erhitzung von empfindlichem Knochenmaterial zu erzeugen, um unter allen Umständen Thermonekrose zu verhindern. Dazu müsste auch der Verschleiss und damit das Stumpfwerden des Trennwerkzeugs frühzeitig sicher erkannt werden können.

Es ist Aufgabe der vorliegenden Erfindung, ein Trennwerkzeug bzw. ein Sägeblatt zu schaffen, welches die genannten Nachteile überwindet und insbesondere eine reduzierte Reibung und Erwärmung des durchtrennten Materials ergibt.

Die erfindungsgemässe Aufgabe wird gelöst durch ein Sägeblatt mit der kombinierten Wirkung der Merkmale gemäss Anspruch 1. Die relativ weiche Edelmetallbeschichtung ergibt eine geglättete Oberfläche mit leicht schmierender Wirkung, wodurch die Reibung vor allem in Knochengewebe stark reduziert wird. Durch die verjüngte Blattdicke im relativ grossen nichtschneidenden Bereich wird hier die Reibung des Sägeblatts am durchtrennten Material stark reduziert, womit es erst möglich und sinnvoll wird, eine weiche Beschichtung einzusetzen. Eine weiche Oberflächenbeschichtung auf dem schneidenden, harten Teil des Zahnbereichs würde sofort abgerieben und wäre damit sinnlos. Da jedoch vergleichsweise der nichtschneidende Bereich flächenmässig sehr gross und der schneidende Zahnbereich sehr klein ist, ergibt die erfindungsgemässe Reibungsverminderung durch die weiche Edelmetallbeschichtung auf dieser grossen Fläche eine wesentliche Reduktion der Gesamtreibung beim Sägen und damit auch eine entsprechend erheblich reduzierte Erwärmung z.B. von empfindlichem Knochenmaterial. Zudem ist es erst durch Einsatz eines biologisch neutralen bzw. körperverträglichen Edelmetalls möglich, einen minimen Abrieb der schmierend wirkenden Schicht zu erzeugen und zuzulassen, da dieser keine schädlichen Folgewirkungen in Knochen und Körper des Patienten hervorruft.

Die erfindungsgemässe Beschichtung steht in vollständigem Gegensatz zu bisher bekannten Beschichtungen, z.B. mit Chrom, welche möglichst hart gewählt werden und bei denen absolut kein Abrieb zulässig ist. Bei bekannten Hartmetallbeschichtungen wie mit Chrom besteht zudem noch eine viel grössere Gefahr. Solche Filme können infolge Beanspruchung bei Sterilisationsprozessen und beim Sägen leicht abblättern. In einem Knochenschnitt zurückbleibende kleine Bruchstücke solcher harter Beschichtungen können sich im Heilungsprozess aber sehr negativ auswirken.

Die weiteren abhängigen Ansprüche betreffen vorteilhafte Weiterbildungen der Erfindung mit besonders geeigneten Sägeblattformen, Materialien in sehr dünnen Schichten und rationelle Herstellverfahren, welche die Trennwirkung weiter steigern und die Erwärmung reduzieren. Um Abnützungsspuren besondes gut zu erkennen, kann sich die Oberflächenbeschichtung optisch deutlich vom Blattsub-stratmaterial unterscheiden und mit Vorteil matt ausgeführt sein. Eine auch aus anderen Gründen vorteilhafte reine Goldbeschichtung ergibt eine besonders deutliche Erkennbarkeit der Abnützung und damit auch des Stumpf-werdens des Trennwerkzeugs.

Mit einer erfindungsgemässen reinen Goldbeschichtung werden auch vorhandene Poren und Unebenheiten des Grundmaterials geglättet und ausgeglichen und eine homogene, gut haftende Oberflächenschicht mit hoher Gleiteigenschaft erzeugt. Ausserdem hat eine Goldbeschichtung bei Spezial-Stahlsorten, wie z.B. HochleistungsSchnellstahl, den Vorteil, eine Oxydation zu verhindern, wodurch es erst möglich wird, solche speziell harten Stähle einwandfrei steril einsetzen zu können.

Eine gut sichtbare, beispielsweise aus Gold bestehende, sehr dünne Edelmetallbeschichtung für chirurgische Sägeblätter lässt durch den Kontakt mit dem zu durchtrennenden Knochen Abnutzungsspuren in der Beschichtung erkennbar werden, so dass der Chirurg aus der Anzahl und Stärke solcher Abnutzungsspuren den Hinweis erhält, dass das betreffende Sägeblatt nicht mehr benutzt werden sollte, weil es stumpf geworden ist und auch bezüglich Sterilität nicht mehr den Anforderungen genügt. Auf diese Weise kann auch verhindert werden, dass unbeabsichtigt oder aus Sparsamkeitsgründen Patienten mit nicht mehr einwandfreiem Werkzeug behandelt werden. Durch Arbeiten mit einem stumpfen Werkzeug besteht die Gefahr, dass ein erhöhter Schneiddruck ausgeübt wird, was zur Entstehung einer hohen Friktonswärme führt. Bei Anwendung in der Chirurgie hat diese Friktionswärme Thermo-Nekrosen, d.h. den Zelltod des Knochengewebes zur Folge, was eine der gefürchtetsten Komplikationen nach operativen Eingriffen am Knochen darstellt. Das Verhängnisvolle dabei ist, dass der Chirurg bei seiner Arbeit weder sehen noch spüren kann, ob er einen solchen Schaden verursacht, der erst im nachhinein erkennbar wird. Auch können der Chirurg oder eine Operationsschwester selbst bei mikroskopischer Untersuchung rein optisch nicht feststellen, ob ein Sägeblatt stumpf ist. Wegen der Unkontrollierbarkeit der Schneidenschärfe und mangelhafter Schneideigenschaften haben deshalb in letzter Zeit Thermo-Nekrosen deutlich zugenommen. Ausserdem kann der Knochen durch Bearbeitung mit einem stumpfen Werkzeug auch splittern, oder es können durch Bruch des Sägeblatts Teile desselben im Knochen verbleiben. Nachteile analoger Art treten ganz allgemein bei der mechanischen Bearbeitung von Werkstücken mit stumpfen oder nicht optimalen Werkzeugen auf, weil das zu bearbeitende Material dabei zu heiss und dadurch geschädigt wird. Dies kann z.B. die Bearbeitung von Kunststoff-Verbundmaterial erheblich erschweren oder sogar verunmöglichen.

Bei Anwendung eines Sägeblatts oder eines spanenden Werkzeugs in der Chirurgie ist es absolut notwendig, dass das eingesetzte Material, welches bei einem operativen Eingriff mit dem lebendigen Körper in Verbindung gebracht wird, nicht zu Immunreaktionen des lebenden Körpers führt oder auch keine anderen schädlichen Wechselwirkungen hervorruft.

Weitere Einzelheiten und Vorteile der Erfindung ergeben sich aus der nachfolgenden Beschreibung und den Zeichnungen, in denen Ausführungsformen von Sägeblättern beispielsweise dargestellt sind. Es zeigt:
- Fig. 1: ein erfindungsgemässes Chirurgie-Sägeblatt für eine geradlinig oszillierende Trennbewegung
- Fig. 2a, b: ein Sägeblatt für eine oszillierende Bewegung auf einem Kreisbogen in Teilansichten von der Seite und von oben gesehen
- Fig. 3: eine Gesamtansicht des Sägeblatts von Fig. 2
- Fig. 4: Abriebspuren in der Edelmatallbeschichtung
- Fig. 5: ein Sägeblatt beim Durchtrennen eines Knochens
- Fig. 6: ein Sägeblatt mit Ausnehmungen
- Fig. 7: ein Sägeblatt mit geschränkten Zähnen
- Fig. 8: ein Beispiel mit verjüngter Dicke im nichtschneidenden Blattbereich

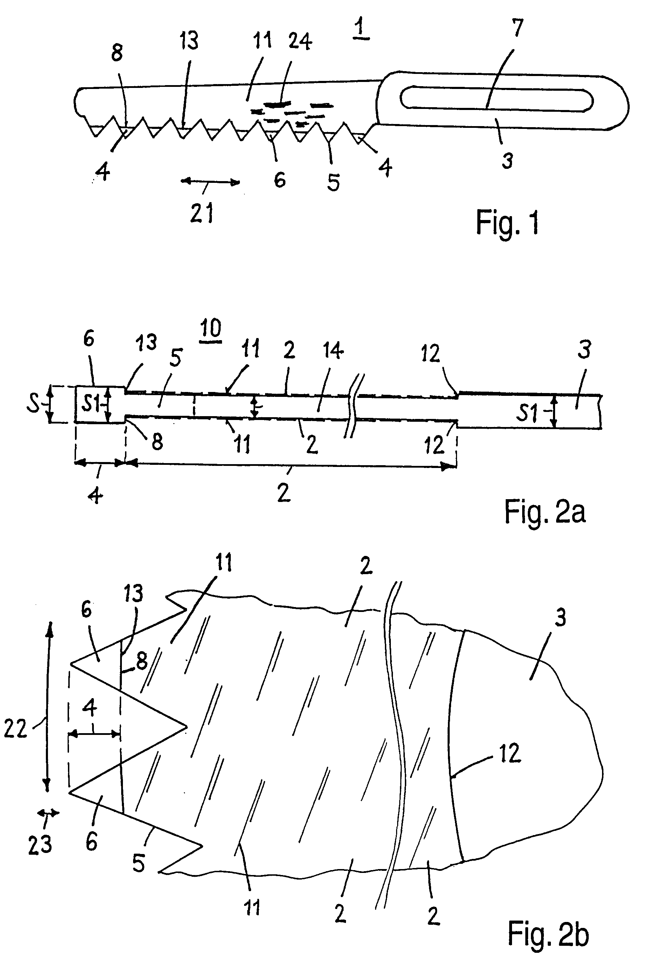

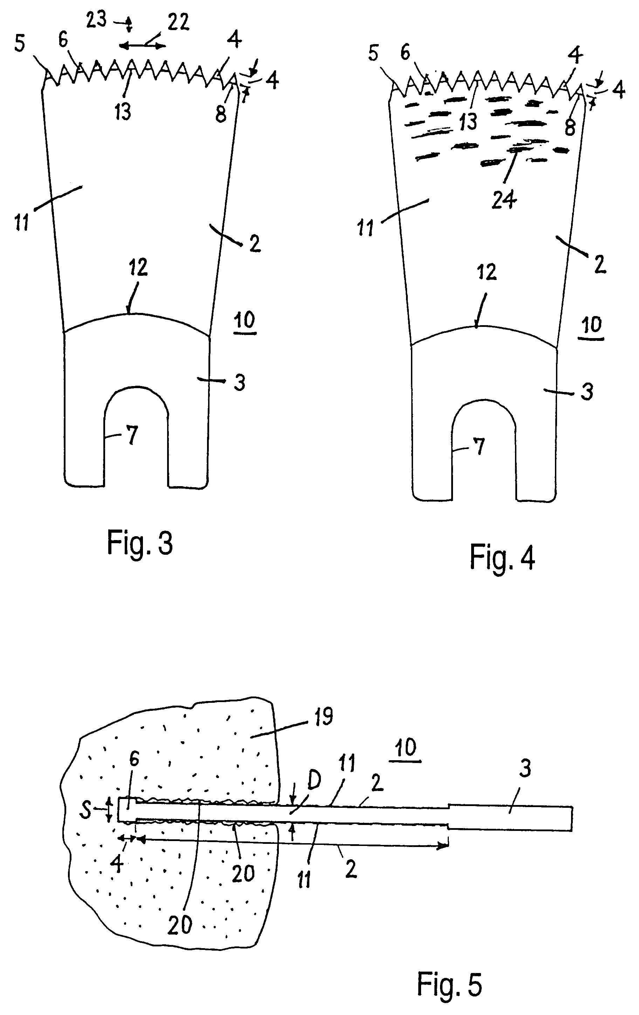

Das in Fig. 1 dargestellte Mikrosägeblatt 1 weist einen Schneidbereich 4 und einen einstückig damit verbundenen Halterungsbereich 3 mit einer Ausnehmung 7 auf, welche zur Befestigung des Sägeblatts an einem Antriebsgerät und zur Erteilung einer im wesentlichen geradlinig oszillierenden Bewegung 21 dient. Das Sägeblatt weist, wie auch im Beispiel von Fig. 2a, 2b, ungeschränkte Sägezähne 5 auf, wobei der von der Linie 8 begrenzte Schneidbereich 4 nur durch die Zahnspitzen 6 in Form von Schneiddreiecken gebildet wird. Die Zahnspitzen im Schneidbereich 4 weisen eine grössere Dicke S1, welche der Schnittbreite S entspricht, auf als der anschliessende nichtschneidende Blattbereich 2 mit der Dicke D. Der nichtschneidende Blattbereich 2 inklusive dem unteren Zahnbereich ist bis zur Begrenzungslinie 13 beidseitig mit einer Edelmetallschicht, vorzugsweise mit Gold oder Platin, dünn beschichtet. Die Figuren 2a, 2b und 3 zeigen als Ganzes und in Teilansichten von der Seite und von oben gesehen als weiteres Beispiel ein Sägeblatt 10 für oszillierende Trennbewegungen im wesentlichen auf einem Kreisbogenabschnitt, z.B. für Schenkelhalsschnitte in der Chirurgie. Die Bewegung des Schneidbereichs 4 der Zähne 5 hat eine Hauptkomponente 22 in tangentialer Richtung mit einer Amplitude von z.B. 1 bis 3 Zahnabständen und eine deutlich kleinere Vertikalkomponente 23 in radialer Richtung. Wie aus Fig. 2a hervorgeht, weisen hier der Halterungsteil 3 und der schneidende Teil 6 der Zähne, welcher durch die Linie 8 begrenzt ist, eine höhere Materialstärke S1 von z.B. 0.35 mm auf, während der anschliessende nichtschneidende Blattbereich 2 eine geringere Materialstärke D von z.B. nur 0.25 mm aufweist. Die Edelmetallbeschichtung 11 überdeckt hier beidseitig den ganzen nichtschneidenden Blattbereich 2, begrenzt durch die Linie 13, welche hier der Schneidbegrenzung 8 entspricht, und gegen die Halterung hin durch die Begrenzungslinie 12. Bevorzugt nehmen die Schneiddreiecke der Zahnspitzen 6 nur etwa 30 bis 50% der Zahnhöhe ein, was bei einer Zahnhöhe von beispielsweise 1 mm eine Schneiddreieckhöhe von 0.3 bis 0.5 mm ergibt. In der Chirurgie können mit derartigen Mikrosägeblättern mit sehr hohen Frequenzen von z.B. 20 000 bis 60 000 Hüben/Min. mit kleiner Amplitude sehr feine Trennschnitte erreicht werden. Anderseits steigen jedoch mit den hohen Frequenzen auch Reibung und Erwärmung, weshalb der erfindungsgemässe reibungsvermindernde Aufbau mit der Edelmetalloberflächenschicht besonders feine und schonende, präzise Schnitte ermöglicht.

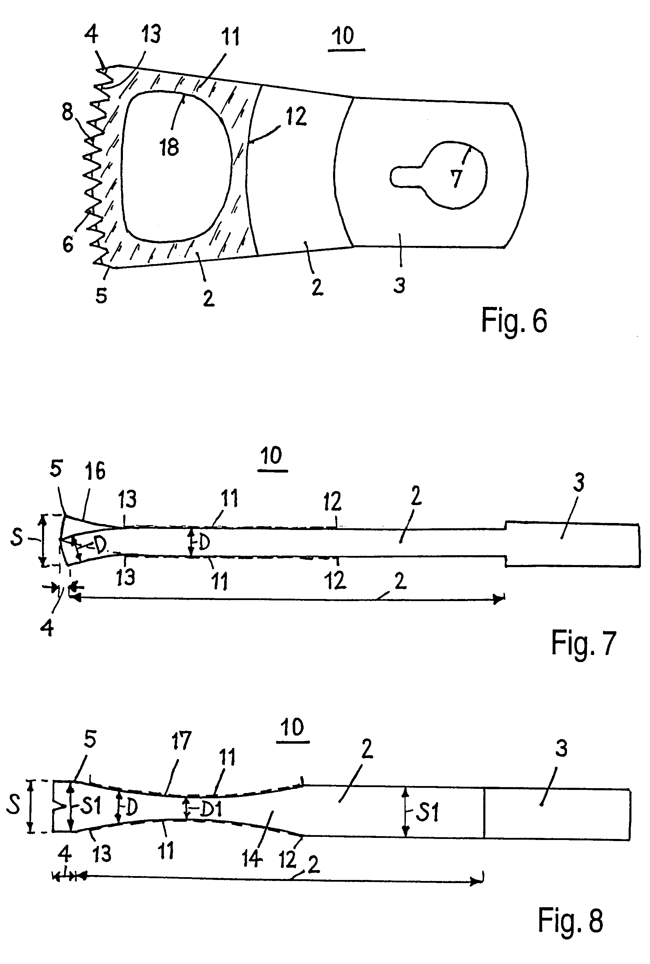

Fig. 7 zeigt ein Sägeblatt mit geschränkten Zähnen 16, welche die gleiche Dicke D aufweisen wie der nichtschneidende Blattbereich 2. Die Schnittbreite S wird durch die wechselseitig auf die eine und die andere Seite geschlagenen Zähne erzeugt. Obwohl solche geschränkten Sägeblätter deutlich schlechtere Trenneigenschaften aufweisen als Sägeblätter mit ungeschränkten Zähnen nach Fig. 1, 2, 5 und 8, können auch hier die Gleiteigenschaften und damit die Trennwirkung deutlich verbessert werden mit der erfindungsgemässen Oberflächenbeschichtung. Fig. 4 zeigt das Sägeblatt von Fig. 2 und 3 nach Gebrauch. Im dünneren nichtschneidenden Blattbereich 2 ist die Edelmetallbeschichtung 11 stellenweise striemenartig abgetragen mit Abriebspuren 24, was anzeigt, dass das Sägeblatt wegen Stumpfheit nicht mehr verwendet werden darf.

Die Funktion des Sägeblatts wird weiter illustriert anhand von Fig. 5, welche einen Knochen 19 während des Trennvorgangs mit einem ungeschränkten Sägeblatt 10 zeigt. Die durchtrennten, relativ grossen Knochenschnittflächen 20 beidseits des Sägeblatts reiben dabei unvermeidbar auf dem nichtschneidenden Blattbereich 2. Diese Reibung wird wie erläutert durch die Oberflächenbeschichtung 11 und die geringere Dicke D wesentlich reduziert. Damit wird auch das Sägeblatt weniger stark beansprucht, wodurch die Bruchgefahr des Blattes sinkt. Dies wiederum ermöglicht es, noch feinere, präzisere und schonendere Mikrosägeblätter einzusetzen. Fig. 6 zeigt ein Sägeblatt mit einer Ausnehmung 18 im nichtschneidenden Bereich 2 nahe der Verzahnung 5, welche die Reibung ebenfalls weiter reduziert.

Fig. 8 zeigt ein weiteres Beispiel eines ungeschränkten Sägeblatts, welches im Schneidbereich 4 und ab der Linie 12 im Blattbereich 2 und im Halterungsbereich 3 die gleiche Dicke S1 aufweist, welche wiederum der Schnittbreite S entspricht. Anschliessend an den Schneidbereich 4 weist das Sägeblatt jedoch eine konkave Verjüngung 17 auf mit einer bis zu einem Minimalwert D1 reduzierten Dicke D. Die Edelmetallbeschichtung ist hier wie auch in Fig. 7 nicht ganz bis zum Schneidbereich 4 hingezogen, sie endet vielmehr vorher an den Begrenzungslinien 13 und 12.

Die Formgebung des Sägeblatts kann durch Laser- oder Wasserstrahlschneiden oder auch durch konventionelle mechanische Bearbeitungsverfahren erfolgen. Ein besonders rationelles Verfahren, z.B. für ein Sägeblatt nach Fig. 2, stellt eine Formgebung durch Abtragen von Blattmaterial 14 mittels Ätzprozess und einer Maske dar, welcher den nichtschneidenden Blattbereich 2 mit Begrenzungslinien 12, 13 offen lässt. Dabei wird die Materialdicke von S1 auf D reduziert. Anschliessend wird mit der gleichen Maske im Blattbereich 2 elektrochemisch bzw. galvanisch eine dünne, glättend wirkende Goldoberflächenschicht 11 aufgetragen. Die Edelmetallbeschichtung wird dünn aufgetragen, z.B. auch durch Vakuumbedampfen, vorzugsweise deutlich unter 1 µ, z.B. in einer mittleren Schichtstärke von 10 - 100 nm, was überdies auch kostengünstig ist. Die Beschichtung wird mit Vorteil matt ausgebildet, um auch einen Blendeffekt beim Operieren zu vermeiden.

Die erfindungsgemässen Trennwerkzeuge eignen sich besonders für medizinische Anwendungen, sie sind aber mit analogen Vorteilen auch in anderen, technischen Gebieten einsetzbar.

## Patentansprüche

1. Sägeblatt für oszillierend oder rotierend ausgeführte Trennschnitte mit einem Schneidbereich, einem nichtschneidenden Blattbereich und einem Halterungsbereich, insbesondere mit Sägezähnen als Schneidbereich und zur Anwendung in der Knochenchirurgie, wobei die Dicke D des Sägeblatts (1, 10) im nichtschneidenden Blattbereich (2) mindestens stellenweise kleiner ist als die Schnittbreite S des Schneidbereichs (4) und im nichtschneidenden Blattbereich beide Seiten des Sägeblatts mindestens stellenweise eine Oberflächenbeschichtung (11) aus einem biologisch neutralen Edelmetall aufweisen und wobei die Edelmetallbeschichtung (11) weicher ist als das Blattsubstratmaterial (14).

2. Sägeblatt nach Anspruch 1, dadurch gekennzeichnet, dass sich die Oberflächenbeschichtung (11) optisch vom Blattsubstratmaterial (14) unterscheidet.

3. Sägeblatt nach Anspruch 1, dadurch gekennzeichnet, dass die Oberflächenbeschichtung (11) den ganzen nichtschneidenden Blattbereich (2) überdeckt und dass der Schneidbereich (4) nicht überdeckt ist.

4. Sägeblatt nach Anspruch 1, dadurch gekennzeichnet, dass Zahnspitzen (6) als Schneidbereich eine grössere Materialdicke (S1) aufweisen als der anschliessende nichtschneidende Blattbereich inklusive Oberflächenbeschichtung (D).

5. Sägeblatt nach Anspruch 1, dadurch gekennzeichnet, dass im nichtschneidenden Blattbereich geschlossene Ausnehmungen (18) vorgesehen sind und dass die Oberflächenbeschichtung vom schneidenen Zahnbereich her mindestens bis zu den Ausnehmungen reicht.

6. Sägeblatt nach Anspruch 1, gekennzeichnet durch eine vakuumbeschichtete Edelmetalloberfläche.

7. Sägeblatt nach Anspruch 1, gekennzeichnet durch eine galvanisch aufgebrachte Edelmetalloberflächenschicht

8. Sägeblatt nach Anspruch 1, dadurch gekennzeichnet, dass die Oberflächenbeschichtung matt ist.

9. Sägeblatt nach Anspruch 1, dadurch gekennzeichnet, dass die mittlere Stärke der Edelmetallbeschichtung weniger als 1 µ beträgt.

10. Sägeblatt nach Anspruch 1, dadurch gekennzeichnet, dass die Stärke der Edelmetallbeschichtung zwischen 10 und 100 nm liegt.

11. Sägeblatt nach einem der vorangehenden Ansprüche, dadurch gekennzeichnet, dass die Edelmetallschicht aus Gold besteht.

12. Sägeblatt nach Anspruch 1, dadurch gekennzeichnet, dass die Edelmetallschicht aus Platin besteht.

13. Sägeblatt nach Anspruch 1, dadurch gekennzeichnet, dass die Stärke der Edelmetallbeschichtung so bemessen ist, dass infolge Abrieb der Beschichtung durch Gebrauch blanke Stellen (24) des Substratmaterials deutlich sichtbar sind bei beginnendem Stumpfwerden des Schneidbereichs (4).

14. Verfahren zur Herstellung eines Sägeblatts nach Anspruch 1, wobei zur Formgebung mittels eines Ätzprozesses Blattmaterial (14) abgetragen und anschliessend die Edelmetalloberflächenschicht (11) galvanisch aufgebracht wird.

## Claims

1. A saw blade for oscillatingly or rotatingly designed separating cuts with a cutting region, with a non-cutting blade region and with a mounting region, in particular with saw teeth as a cutting region and for application in bone surgery, wherein the thickness D of the saw blade (1, 10) in the non-cutting blade region (2) at least at locations is smaller than the width of cut S of the cutting region (4), and in the non-cutting blade region both sides of the saw blade at least at locations have a surface coating (11) of a biologically neutral precious metal and wherein the precious metal coating (11) is softer than the blade substrate material (14).

2. A saw blade according to claim 1, characterised in that the surface coating (11) optically differentiates itself from the blade substrate material (14).

3. A saw blade according to claim 1, characterised in that the surface coating (11) covers the whole non-cutting blade region (2) and that the cutting region (4) is not covered.

4. A saw blade according to claim 1, characterised in that tooth tips (6) as a cutting region have a larger material thickness (S1) than the connecting non-cutting blade region including the surface coating (D).

5. A saw blade according to claim 1, characterised in that in the non-cutting blade region there are provided closed recesses (18) and that the surface coating from the cutting tooth-region at least reaches up to the recesses.

6. A saw blade according to claim 1, characterised by a vacuum coated precious metal surface.

7. A saw blade according to claim 1, characterised by a galvanically deposited precious metal surface layer.

8. A saw blade according to claim 1, characterised in that the surface coating is matt.

9. A saw blade according to claim 1, characterised in that the average thickness of the precious metal coating is smaller than 1 µ.

10. A saw blade according to claim 1, characterised in that the thickness of the precious metal coating is between 10 and 100 nm.

11. A saw tooth according to one of the preceding claims, characterised in that the precious metal layer consists of gold.

12. A saw tooth according to claim 1, characterised in that the precious metal layer consists of platinum.

13. A saw tooth according to claim 1, characterised in that the thickness of the precious metal coating is dimensioned such that as a result of wear of the coating by use naked locations (24) of the substrate material are clearly visible when the cutting region (4) begins to blunten.

14. A method for manufacturing a saw blade according to claim 1, wherein for shaping by way of an etching process blade material is removed and subsequently the precious metal surface layer (11) is galvanically deposited.

## Revendications

1. Lame de scie destinée à exécuter des coupes cisaillées par oscillation ou rotation, comportant une zone de coupe, une zone de lame non coupante et une zone de support, comportant en particulier des dents formant la zone de coupe, destinée à être utilisée en chirurgie des os, l'épaisseur D de la lame de scie (1, 10) dans la zone de lame non coupante (2) étant au moins par endroits inférieure à la largeur de coupe S de la zone de coupe (4), les deux côtés de la lame de scie présentant dans la zone de lame non coupante au moins par endroits un revêtement de surface (11) composé d'un métal précieux neutre du point de vue biologique, le revêtement de métal précieux (11) étant ainsi plus mou que le matériau du substrat de la lame (14).

2. Lame de scie selon la revendication 1, caractérisée en ce que le revêtement de surface (11) se distingue optiquement du matériau du substrat de la lame (14).

3. Lame de scie selon la revendication 1, caractérisée en ce que le revêtement de surface (11) recouvre l'ensemble de la zone non coupante de la lame (2) et en ce que la zone de coupe (4) n'est pas recouverte.

4. Lame de scie selon la revendication 1, caractérisée en ce que les pointes des dents (6) composant la zone de coupe ont une épaisseur de matériau (S1) supérieure à celle de la zone non coupante suivante de la lame, y compris le revêtement de surface (D).

5. Lame de scie selon la revendication 1, caractérisée en ce que la zone non coupante de la lame comporte des évidements fermés (18), le revêtement de surface s'étendant de la zone de coupe des dents au moins jusqu'aux évidements.

6. Lame de scie selon la revendication 1, caractérisée par une surface de métal précieux à métallisation sous vide.

7. Lame de scie selon la revendication 1, caractérisée par une couche de surface de métal précieux appliquée par galvanisation.

8. Lame de scie selon la revendication 1, caractérisée en ce que le revêtement de surface est mat.

9. Lame de scie selon la revendication 1, caractérisée en ce que l'épaisseur moyenne du revêtement de métal précieux est inférieure à 1 µ.

10. Lame de scie selon la revendication 1, caractérisée en ce que l'épaisseur du revêtement de métal précieux est comprise entre 10 et 100 nm.

11. Lame de scie selon l'une des revendications précédentes, caractérisée en ce que la couche de métal précieux est composée d'or.

12. Lame de scie selon la revendication 1, caractérisée en ce que la couche de métal précieux est composée de platine.

13. Lame de scie selon la revendication 1, caractérisée en ce que l'épaisseur du revêtement de métal précieux est dimensionnée de sorte que par suite de l'usure du revêtement en service, les endroits dénudés (24) du matériau du substrat sont nettement visibles lors du début de l'émoussage de la zone coupante (4).

14. Procédé de fabrication d'une lame de scie selon la revendication 1, l'étape de formage comportant l'élimination de matériau de la lame (14) par l'intermédiaire d'un procédé de décapage avant l'application de la couche de surface de métal précieux (11) par galvanisation.
